# EUROPEAN PATENT SPECIFICATION

(11) **EP 3 471 609 B1**
(45) Date of publication and mention of the grant of the patent: **24.04.2024**
(21) Application number: 17729899.9
(22) Date of filing: 16.06.2017
(51) Int. Cl.: A61B 5/11

(54) **QUANTITATIVE SEISMOCARDIOGRAPHY**
QUANTITATIVE SEISMOKARDIOGRAFIE
SÉISMOCARDIOGRAPHIE QUANTITATIVE

(30) Priority: 16.06.2016 EP 16174743
(43) Date of publication of application: 24.04.2019
(73) Proprietor: Acarix A/S, 2800 KGS Lyngby (DK)
(72) Inventor: SCHMIDT, Samuel Emil, 9210 Aalborg SØ (DK); SØGAARD, Peter, DK-2300 København S (DK); STRUIJK, Johannes Jan, DK-9575 Terndrup (DK)
(74) Representative: Brann AB
(86) International application number: PCT/EP2017/064834
(87) International publication number: WO 2017/216374

(56) References cited:
- US-A1- 2003 233 034
- US-A1- 2008 021 336
- US-A1- 2011 066 042
- US-A1- 2013 109 989
- US-A1- 2015 038 856
- US-A1- 2015 342 466
- DI RIENZO M ET AL: "Wearable seismocardiography: Towards a beat-by-beat assessment of cardiac mechanics in ambulant subjects", AUTONOMIC NEUROSCIENCE: BASIC AND CLINICAL, ELSEVIER, AMSTERDAM, NL, vol. 178, no. 1, 9 May 2013 (2013-05-09), pages 50-59, XP028734850, ISSN: 1566-0702, DOI: 10.1016/J.AUTNEU.2013.04.005
- PANDIA KEYA ET AL: "A frequency domain analysis of respiratory variations in the seismocardiogram signal", ENGINEERING IN MEDICINE AND BIOLOGY SOCIETY (EMBC), 2013 34TH ANNUAL INTERNATIONAL CONFERENCE OF THE IEEE, IEEE, 3 July 2013 (2013-07-03), pages 6881-6884, XP032489567, ISSN: 1557-170X, DOI: 10.1109/EMBC.2013.6611139 [retrieved on 2013-09-25]

## Description

### Technical field of the Invention

The present invention generally relates to techniques for diagnostic purposes relating to cardiovascular function, and in particular to techniques for assisting in diagnosing systolic and diastolic dysfunction that could lead to heart failure.

### Background of the Invention

Diastolic dysfunction is a frequent occurring heart defect in persons older than 45 years. Diastolic dysfunction can lead to severe heart failure and is associated with increased mortality. Current methods for diagnosis of diastolic dysfunction are complicated and expensive, which typically means that they are used only on patients with high risk of diastolic dysfunction.

Seismocardiography (SCG) is the analysis of sub-audible low-frequency vibrations at the chest wall caused by the beating heart. More generally, SCG relates to non-invasive measurement of accelerations in the chest wall produced by myocardial movement. Heart sounds are audible components of the chest wall vibrations that typically are above 40-60 Hz, while SCG vibrations typically are below 5 Hz.

SCG is typically measured using an accelerometer. However, when an accelerometer is used, both low frequency SCG components and audible components are simultaneously sampled. The SCG components and the audible components reveal different cardiovascular functions, thus enabling different approaches to diagnosing a cardiovascular function. For example, SCG is typically suitable for estimation of time intervals between features in the cardiac cycle, while heart sounds are appropriate for detection of murmurs caused by flow disturbances.

When using an accelerometer, the heart sounds or audio components in the accelerometer signal are dominated by the high intensity of the low-frequency vibrations, or SCG waves. If the accelerometer signal is high pass filtered, for example with a lower cutoff of 50 Hz, the heart sounds are revealed. In the heart sound, the most dominating sounds are the first heart sound (S1) and second heart sounds (S2), which are related to the mitral valve closure (MC) and the aortic valve closure (AC), respectively.

US2013109989 discloses a method and apparatus for obtaining and processing ballistocardiograph data to determine a physiological condition of a subject. Ballistocardiograph data indicative of heart motion of the subject measured along a plurality of spatial axes by a sensor device which may comprise a three-axis accelerometer. The ballistocardiograph data is processed to determine processed data indicative of heart motion of the subject. Indications of physiological condition are determined based at least in part on the processed data. Processing may comprise aggregation of multidimensional data, determining magnitude of heart motion and derivative thereof, determining a thrust summation, determining an index value, outputting a report based on an index value, etc. Processing may be informed by operator input, such as a time window of interest or indications of interest.

A problem for General Practitioners is that heart failure shares symptoms with other common diseases, such as respiratory disease. Thus, there is a need for a reliable or accurate tool that can assist in diagnosing or determining a probability of heart failure.

### Object of the Invention

An object of the present invention is to provide an improved tool for quantifying the function of a beating heart. It is also an object to provide an improved technology for identifying heart failure.

### Summary of the Invention

The invention is set out in the claims.

### Summary of the Disclosure

According to a first aspect, the aforementioned objects are accomplished by a method for quantifying the function, or cardiovascular function, of a beating heart. The method comprises: obtaining a plurality of segments of a signal recorded with an accelerometer, e.g. placed on the chest of a person for measuring accelerations and vibrations of the chest wall of the person caused by myocardial movement, wherein each segment covers, or corresponds to, a cardiac cycle. The method further comprises: aligning the plurality of segments, determining a mean segment based on the plurality of segments, and filtering the plurality of segments prior to determining the mean segment, or filtering the mean segment, with a band-pass filter having a lower cutoff frequency below 5 Hz, preferably below 1 Hz, and an upper cut-off frequency in the range 100-500 Hz. The method further comprises: determining a first temporal feature in the filtered mean segment, determining a measure based on at least one of the signal strength, or amplitude, of the first temporal feature and the location in time of the first temporal feature, and providing output information based on the determined measure.

Here, and throughout these specifications, quantifying the function, or cardiovascular function, is understood to be limited to quantifying, or determining an indication of, an abnormal cardiovascular function, condition or structure, and/or a cardiovascular disorder. Thus, quantifying the function is understood to encompass quantifying, or determining an indication of, a heart disease relating to myocardial performance, such as heart failure.

Quantifying the function, or cardiovascular function, is understood to not include quantifying, or determining an indication of, fitness, such as cardiovascular or cardiorespiratory fitness. Thus, quantifying the function is understood to not encompass quantifying, or determining an indication of, aerobic fitness, such as maximal oxygen consumption or uptake (VO₂ Max). Here, fitness is understood to be related to a normal cardiovascular function, and to be disassociated with, an abnormal cardiovascular function, condition or structure, or a cardiovascular disorder.

The specified band-pass filtering has the effect that the temporal feature is determined from mean segment including both low-frequency SCG components and audible components, as compared to when only low-frequency SCG components are considered. It has been found the specified band-pass filtering contributes to a reliable, and thus improved, quantifying of heart failure.

Throughout these specifications, a temporal feature may correspond to a feature or stage in a cardiac cycle. A temporal feature may correspond to a peak, valley, local extremum, local minima, local maxima, maximal change, maximal increase, or maximal decrease of the filtered mean segment. A measure may, throughout these specifications, correspond to, or be based on a signal strength or an amplitude, or a difference in time. The signal strength or amplitude of a temporal feature may correspond to an acceleration affecting the accelerometer. Signal strength of a temporal feature is here, and throughout these specifications, understood to encompass a signal sample or a signal value of the temporal feature. The amplitude may be determined relative to the mean signal in the mean segment. An amplitude is understood to encompass a peak value, or the extreme value of a temporal feature, such as a local maxima or minima.

The accelerometer may comprise a piezoelectric element. The signal may represent a voltage generated by the piezoelectric element. Thus, the signal strength or amplitude of a temporal feature may represent a voltage value for the temporal feature.

According to a second aspect, the objects are achieved by system for quantifying the function, or cardiovascular function, of a beating heart. The system comprises:(A) an accelerometer, e.g. configured to be placed on the chest of a person for measuring accelerations and vibrations of the chest wall of the person caused by myocardial movement, and (B) a processor operatively connected to the accelerometer. The processor is configured to: obtain a plurality of segments of a signal recorded with the accelerometer, wherein each segment covers, or corresponds to, a cardiac cycle. The processor is also configured to: align the plurality of segments, determine a mean segment based on the plurality of segments, and filter the plurality of segments prior to determining the mean segment, or filter the mean segment, with a band-pass filter having a lower cutoff frequency below 1 Hz and an upper cut-off frequency in the range 100-500 Hz. The processor is further configured to: determine a first temporal feature in the mean segment, determine a measure based on at least one of the signal strength, or amplitude, of the first temporal feature and the location in time of the first temporal feature, and provide output information based on the determined measure.

In the above aspects, to obtain a plurality of segments of a signal may comprise: obtaining the signal and forming the plurality of segments from the signal.

According to a third aspect, the objects are achieved by a system for quantifying the function, or cardiovascular function. The system comprises: an accelerometer, e.g. configured to be placed on the chest of a person for obtaining a signal representing accelerations and vibrations of the chest wall of the person caused by myocardial movement, and a segmentation module for forming a plurality of segments from the signal, wherein each segment covers, or corresponds to, a cardiac cycle. It further comprises: an align module for aligning the plurality of segments, a first calculation module for determining a mean segment based on the plurality of segments, and a filter module for filtering the plurality of segments prior to determining the mean segment, or for filtering the mean segment, with a band-pass filter having a lower cutoff frequency below 1 Hz and an upper cut-off frequency in the range 100-500 Hz. The system also comprises: a second calculation module for determining a first temporal feature in the mean segment and, a third calculation unit for determining a measure based on at least one of the signal strength, or amplitude, of the first temporal feature and the location in time of the first temporal feature, and an output module for providing output information based on the determined measure.

According to a fourth aspect, the objects are achieved by a computer program product for being used in a system for quantifying heart failure comprising: (A) an accelerometer, e.g. for being placed on, or configured to be placed on, the chest of a person for measuring accelerations and vibrations of the chest wall of the person caused by myocardial movement, and (B) a processor operatively connected with the accelerometer. The computer program product comprising program code instructions configured to, when executed by the processor of the system, cause the processor to: obtain a signal with the accelerometer, and forming a plurality of segments from the signal, wherein each segment covers, or corresponds to, a cardiac cycle. The program code instructions further causes the processor to: align the plurality of segments, determine a mean segment based on the plurality of segments, and filter the plurality of segments prior to determining the mean segment, or filter the mean segment, with a band-pass filter having a lower cutoff frequency below 1 Hz and an upper cut-off frequency in the range 100-500 Hz. The program code instructions are further configured to cause the processor to: determine a first temporal feature in the mean segment, determine a measure based on at least one of the signal strength, or amplitude, of the first temporal feature and the location in time of the first temporal feature, and provide output information based on the determined measure.

According to a fifth aspect, the objects are achieved by a non-transient memory on which a computer program product according to the fourth aspect is stored.

In the different aspects above, the output information may represent the actual determined measure. Alternatively, the output information may represent a score based on the determined measure. The output information may indicate function, cardiovascular function, heart failure, or risk for heart failure or another abnormal condition, for example as one or more numerical values. Additionally or alternatively, the aligning may be performed prior to the filtering, and the filtering may be performed prior to determining the mean segment.

In the different aspects above, the step of filtering the plurality of segments prior to determining the mean segment, or filtering the mean segment, may be omitted, in particular if a microphone is used in addition to the accelerometer. Additionally or alternatively, the accelerometer placed on the chest of a person for measuring accelerations and vibrations of the chest wall of the person caused by myocardial movement may be replaced with a accelerometer for measuring accelerations and vibrations caused by myocardial movement, and the accelerometer may be configured to be placed on the chest of a person for measuring accelerations and vibrations of the chest wall of the person, or for being inserted into the body of the person, for example as an implantable device, or inside a blood vessel, such a blood vessel at the heart.

In the method of the first aspect, the accelerometer may be placed on the chest of a person and attached to the skin of the person by an adhesive for measuring the accelerations and vibrations. The systems of the second, third and fourth aspects may further comprise an adhesive patch configured for supporting the accelerometer, or a housing described below, and for being attached to the skin of the person. By attaching the accelerometer, or housing, to the skin, the quality of the recorded signals is improved.

### Detailed description

The different aspects described above may be modified as described below.

The step of obtaining a plurality of segments of a signal recorded with an accelerometer comprises recording a signal with an accelerometer, e.g. placed on the chest of a person for measuring accelerations and vibrations of the chest wall of the person caused by myocardial movement, wherein the signal may be - recorded over a period of time covering a plurality of cardiac cycles of the person. The step further comprises:
dividing the recorded signal into the plurality of segments, wherein each segment may cover a single cardiac cycle.

The accelerometer may be placed on the front of the chest of the person. The accelerometer being placed on the chest of a person means that it is placed on the outside and not on the inside of the body. This has the advantage of a simple application that does not require any chirurgical skills and that it can be used in non-sterile environments.

According to one aspect of the invention, obtaining a plurality of segments of a signal recorded with an accelerometer comprises recording the signal with the accelerometer, e.g. placed on the chest of a person for measuring accelerations and vibrations of the chest wall of the person caused by myocardial movement, and recording an audio signal with a microphone placed on the chest of the person simultaneously to recording the signal with the accelerometer. The microphone is configured for measuring cardiovascular sounds, or sounds generated by the beating heart. Obtaining a plurality of segments further comprises identifying a plurality of heart sounds in the audio signal, wherein each heart sound relates to a single cardiac cycle, and dividing the recorded signal into the plurality of segments based on the identified plurality of heart sounds. According to another aspect of the invention, obtaining a plurality of segments of a signal recorded with an accelerometer comprises: recording the signal with the accelerometer, e.g. placed on the chest of a person for measuring accelerations and vibrations of the chest wall of the person caused by myocardial movement, and filtering the signal to obtain an audio signal. Obtaining a plurality of segments further comprises identifying a plurality of heart sounds in the audio signal, wherein each heart sound relates to a single cardiac cycle, and dividing the recorded signal into the plurality of segments based on the identified plurality of heart sounds. The filtering may comprise a high-pass filtering having lower cut-off frequency in the range 40-80 Hz, or approximately equal to 50 Hz or 65 Hz.

Here, the plurality of heart sounds may be the first heart sound (S1). Alternatively, the plurality of heart sounds may be the second heart sound (S2). Throughout these specifications, a microphone is understood as a transducer that converts sound into an electrical signal.

The aligning the plurality of segments may comprise: determining a heart sound in each of the plurality of segments, and aligning the plurality of segments by the determined heart sound of each segment. The heart sound may be the first heart sound (S1) or the second heart sound (S2). The first heart sound (S1) may correspond to the closing of the atrioventricular valves. The second heart sound (S2) may correspond to the closing of the semilunar valves. It has been found that the specified aligning, together with the specific filtering, contributes to a reliable quantifying of heart failure.

Further, determining a measure may comprise: determining the signal strength, or amplitude, of the first temporal feature. The first temporal feature may correspond to: the aortic valve opening (AO) of a heart cycle, the atrial systole (AS) of a heart cycle, the isometric contraction (IM) of a heart cycle, or the rapid ventricular ejection or rapid emptying event (RE) of a heart cycle. It has been found that these features are particularly suitable for quantifying heart failure. A possible explanation is that heart failure cause weaker cardiac contraction, and thus influences the signal strength.

The method according to the first aspect may further comprise: determining a second temporal feature in the filtered mean segment, and wherein determining a measure is further based on at least one of the signal strength, or amplitude, of the second temporal feature and on the location in time of the second temporal feature. By having two or more temporal features, additional measures can be used, thus contributing to an improved technology for identifying heart failure.

The measure may further be based on the location in time of the first temporal feature and on the location in time of the second temporal feature. Determining a measure may comprise: determining the difference between the location in time of the first temporal feature and the location in time of the second temporal feature, wherein the measure is based on the determined difference. Alternatively, determining a measure may comprise: determining the time interval between the first temporal feature and the second temporal feature, wherein the measure is based on the determined time interval.

Alternatively or additionally, the measure may be based on the signal strength, or amplitude, of the first temporal feature and on the signal strength, or amplitude, of the second temporal feature. The signal strength, or amplitude, of the first temporal feature may be normalized by the signal strength, or amplitude, of the second temporal feature. Determining a measure may comprise: determining the difference or ratio between the signal strength, or amplitude, of the first temporal feature and the signal strength, or amplitude, of the second temporal feature, wherein the measure is based on the determined difference or ratio.

The first temporal feature may correspond to the mitral valve closure (MC) and the second temporal feature may correspond to the rapid ventricular ejection or rapid emptying event (RE). Alternatively, the first temporal feature may correspond to the atrial systole (AS) in the cardiac cycle and the second temporal feature may correspond to the mitral valve closure (MC) in the cardiac cycle. Alternatively, the first temporal feature may correspond to the aortic valve closing (AC) and the second temporal feature may correspond to the mitral valve opening (MO). Alternatively, the first temporal feature may correspond to the aortic valve opening (AO) and the second temporal feature may correspond to the aortic valve closing (AC). Alternatively, the first temporal feature may correspond to the mitral valve closure (MC) and the second temporal feature may correspond to the aortic valve opening (AO). It has been found that these features are particularly suitable for quantifying heart failure. A possible explanation is that heart failure cause slower cardiac contraction, and thus influences the timing in the heart cycle.

The method according to the first aspect may further comprise: determining a third temporal feature in the filtered mean segment, and wherein determining a measure is further based on at least one of the signal strength, or amplitude, of the third temporal feature and on the location in time of the third temporal feature. Determining a measure may comprise: determining a first difference between the location in time of the first temporal feature and the location in time of the second temporal feature, wherein the measure is based on the first difference and the signal strength, or amplitude, of the third temporal feature. The first, second, and third temporal feature may correspond to the mitral valve closure (MC), the aortic valve closing (AC), and the rapid emptying event (RE), respectively.

The method according to the first aspect may further comprise: determining a fourth temporal feature in the filtered mean segment, and wherein determining a measure is further based on at least one of the signal strength, or amplitude, of the fourth temporal feature and on the location in time of the fourth temporal feature. Determining a measure may comprise: determining a second difference between the location in time of the second temporal feature and the location in time of the fourth temporal feature, wherein the measure is further based on the second difference. The fourth temporal feature may correspond to the aortic valve opening (AO) .

The method according to the first aspect may further comprise: determining a fifth temporal feature in the filtered mean segment, and wherein determining a measure is further based on at least one of the signal strength, or amplitude, of the fifth temporal feature and on the location in time of the fifth temporal feature. Determining a measure may comprise: determining a third difference between the location in time of the first temporal feature and the location in time of the fifth temporal feature, wherein the measure is further based on the third difference. The fifth temporal feature may correspond to the atrial systole (AS).

The method, or determining the first temporal feature, may further comprise: determining a first point in time in the mean segment corresponding to the onset of a heart sound. Determining the first temporal feature may further comprise: determining the first temporal feature relative to the first point in time. Similarly, determining the second temporal feature may further comprise: determining the second temporal feature relative to the first point in time. The heart sound may be the first heart sound (S1) or the second heart sound (S2). As mentioned above, the first heart sound (S1) may correspond to the closing of the atrioventricular valves and the second heart sound (S2) may correspond to the closing of the semilunar valves.

If the heart sound is the first heart sound (S1), determining a first temporal feature may comprise: determining the first local minima (IM) subsequent to the first point in time, and assigning the first local minima to represent the isovolumic movement (IM), and/or determining the maximum negative deviation (MC) prior to the first local minima (IM) subsequent to the first point in time, and assigning the maximum negative deviation to represent the mitral valve closure (MC). Alternatively or additionally, determining a first temporal feature may comprise: determining the global maxima subsequent to the first point in time, and assigning the global maxima to represent the aortic valve opening (AO), and/or determining the global maxima (RE) subsequent to the global minima (IC) subsequent to the global maxima (AO) subsequent to the first point in time, and assigning the global maxima (RE) subsequent to the global minima to represent the rapid ventricular ejection or rapid emptying event (RE). Here, a global extremum on one side of an event only relate to the local extrema on the same side of the event. It has been found that these definitions are advantageous for determining an indication of heart failure.

The lower cutoff frequency of the band-pass filter may be below 0.5 Hz, 0.2 Hz, or approximately 0.1 Hz. The upper cutoff frequency may be the range of 150-250 Hz, 175-225 Hz, or approximately 200 Hz, or in one of the ranges 100-150 Hz, 150-200 Hz, and 200-250 Hz. According to the invention, the upper cutoff frequency is in the range 100-250 Hz. These frequencies for providing the SCG signal have been found to give reliable results.

The method may further comprise: determining a heart rate of the beating heart. Similarly, the processor may be configured to: determine a heart rate of the beating heart, and the computer program product may comprising program code instructions configured to, when executed by the processor of the system, cause the processor to: determine a heart rate of the beating heart. The heart rate may be determined based on the signal recorded with the accelerometer. The heart rate may indicate the number of contraction of the heart per minute or another suitable period of time.

Determining the measure may further be based on the heart rate. For example, determining the measure may comprise: determining the difference between the location in time of a first temporal feature and the location in time of a second temporal feature and dividing the difference with the heart rate. It is contemplated that by taking the heart rate into account, the measure can be determined more accurately for persons having a high heart rate at rest and for persons that are active or exercising when the signal is recorded with the accelerometer.

The system of the above aspects may comprise: (C) a non-transient memory storing program code instructions that, when executed by the processor, configures the processor to perform the described steps and/or have the described functions.

The system may comprise a smart-phone. The processor and/or the non-transient memory may be integral parts of the smart-phone. Further, the accelerometer may be an integral part of the smart-phone. The system may also comprise a casing or holder for supporting the smart-phone, and the casing or holder may comprise an adhesive patch configured for attaching the casing or holder to the skin of the person. Alternatively to the accelerometer being an integral part of the smart-phone, the accelerometer may form part of an auxiliary unit configured to communicate with the smart-phone by wire or wirelessly, such as a band that can be strapped around the chest of person.

The processor is further configured to: operate the accelerometer to record a signal, e.g. with the accelerometer placed on the chest of a person, wherein the signal is recorded over a period of time covering a plurality of cardiac cycles of the person. The processor is further configured to: divide the recorded signal into a plurality of segments to obtain the plurality of segments of a signal, wherein each segment covers a single cardiac cycle.

According to an aspect of the invention, the system of the above aspects comprises (D) a microphone configured to be placed on the chest of the person for measuring sounds generated by the beating heart, wherein the processor is further operatively connected to the microphone and configured to: operate the accelerometer to record a signal with the accelerometer, e.g. placed on the chest of a person; operate the microphone to record an audio signal with the microphone placed on the chest of a person simultaneously to the signal being recorded with the accelerometer; identify a plurality of heart sounds in the audio signal, wherein each heart sound relates to a single cardiac cycle; and divide the recorded signal into the plurality of segments based on the identified plurality of heart sounds to obtain the plurality of segments.

According to another aspect of the invention, the processor is further configured to:
operate the accelerometer to record a signal, e.g. with the accelerometer placed on the chest of a person; filtering the signal to obtain an audio signal; identify a plurality of heart sounds in the audio signal, wherein each heart sound relates to a single cardiac cycle; and divide the recorded signal into the plurality of segments based on the identified plurality of heart sounds to obtain the plurality of segments. The filtering may comprise a high-pass filter having lower cut-off frequency in the range 40-60 Hz, or approximately equal to 50 Hz.

Here, the plurality of heart sounds may be the first heart sound (S1). Alternatively, the plurality of heart sounds may be the second heart sound (S2).

The system may comprise a housing or cover that supports and encloses or covers the processor. The housing or cover may further enclose or cover at least a portion of, or the whole of, the accelerometer and/or enclose or cover at least a portion of, or the whole of, the microphone.

Providing the output information may further comprise: storing the plurality of segments, the mean segment and/or the measure in the non-transient memory or in the auxiliary non-transient memory. The auxiliary non-transient memory may form part of computer server system, which may be at a remote location.

Providing the output information may further comprise: providing a previously obtained measure and the output information may further be based on the previously obtained measure. The previously obtained measure may be stored in the non-transient memory or in the auxiliary non-transient memory. The output information may be based on the difference between the measure and the previously obtained measure. For example, the output information may be the difference in amplitude between a first heart sound (S1) and a previously obtained heart sound.

Additionally or alternatively, providing the output information may further comprise: providing a previously obtained mean segment and the output information may further be based on the mean segment and the previously obtained mean segment. The previously obtained mean segment may be stored in the non-transient memory or in the auxiliary non-transient memory. The output information may comprise: a graph overlying the mean segment with the previously obtained mean segment. More specifically, the output information may comprise: a graph overlying a portion of the mean segment and the corresponding portion of the previously obtained mean segment. For example, the portion may cover the first heart sound (S1). The graph may be displayed on the screen of abovementioned smart-phone.

The previously obtained measure or previously obtained mean segment may have been determined in the same manner as the measure or the mean segment, or by the same steps as performed for determining the measure or the mean segment. The previously obtained measure or previously obtained mean segment may have been determined at an earlier point in time, such as more than five days or ten days prior to determining the measure or the mean segment.

Further advantages with and features of the different aspects will be apparent from the following description of the drawing.

### Brief description of the drawings

A more complete understanding of the abovementioned and other features and advantages of the present invention will be apparent from the following detailed description of the drawings, wherein:
Fig.1 is a schematic illustration of an embodiment of a system for quantifying the function of a beating heart,
Fig.2 is a flow chart illustrating the basic steps of a method employed in the system described in relation to Fig.1,
Fig.3 is a flow chart illustrating sub-steps and additional steps of a method based on the method described in relation to Fig.2,
Figs.4a and 4b shows a segment that has been filtered by a band-pass filter and to a high-pass filter, respectively,
Fig.5 is a schematic illustration of an embodiment of a system an alternative embodiment for quantifying the function of a beating heart,
Fig.6 is a schematic illustration of alternative sub-steps employed in the system described in relation to Fig.5, and
Figs.7a and b are schematic illustrations of alternative embodiment of a system for quantifying the function of a beating heart.

### Detailed description of drawings

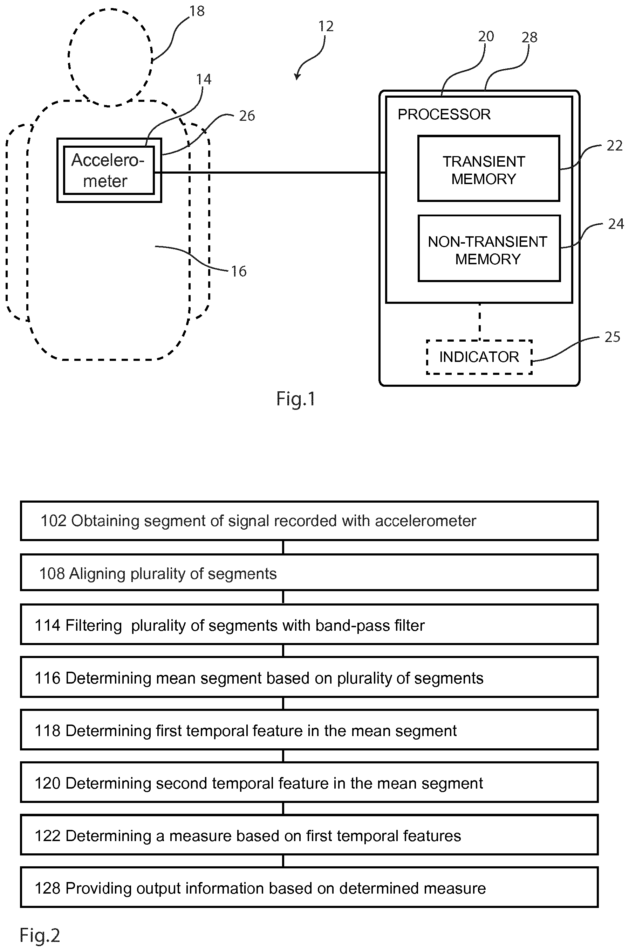

Fig.1 schematically illustrates an embodiment of a system 12 for quantifying heart failure. The system 12 has an accelerometer 14 in the form of a piezoelectric element that can be placed on the chest of a person 18 and for measuring vibrations of the chest wall caused by movements of the heart. A processor 20 is connected with the accelerometer 14. The processor 20 has a transient memory 22 which can store a signal received from the accelerometer 14, and by which it can execute program code instructions. The system 12 comprises a support 26 that supports the accelerometer 14 and a housing 28 that accommodates the processor 20. The system 12 also has a non-transient memory 24 storing program code instructions for the processor 20. For example, the system 12 as a whole can be an integral part of a smart-phone, or all parts except the accelerometer 20 and the support 26 can form part of a smart-phone. In one embodiment, the accelerometer is an integrated accelerometer of a smart-phone.

In one embodiment of the system 12, it additionally has an indicator 25 operatively connected with the processor 20. The indicator 25 can, for example, have an LCD display, or the like, that can display output information from the processor 20, such as a number.

The program code instructions in the non-transient memory 24 cause the processor 20 to perform a method that is shown in Fig.2. The accelerometer is placed on the chest of a person and a signal is recorded. A plurality of segments is then obtained 102, where each segments corresponds to a heartbeat or a cardiac cycle. This is followed by an alignment 108 and a filtering 114. Here, a band-pass filter is employed having a lower cut-off frequency of approximately 0.1 Hz, and an upper cut-off frequency of approximately 200 Hz. Subsequently, a mean segment is determined 118 from the plurality of segments.

With the mean segment formed, a temporal feature is determined 120. The temporal feature in turn is used to determine 122 a measure. Examples of temporal features and measures are described below. Output information is then provided 128 based on the determined measure. In one embodiment, the output information is a number that is displayed on the abovementioned indicator 25.

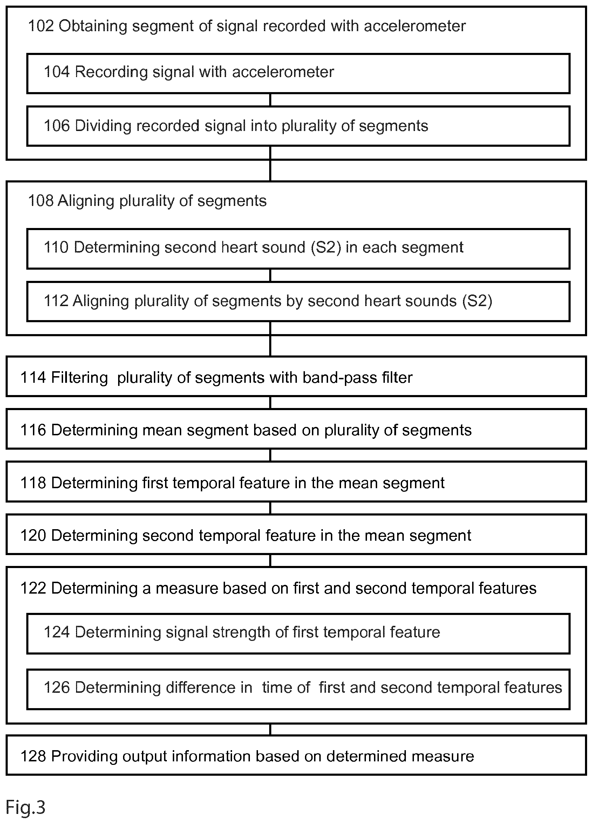

Further details of the method are shown in the flow chart of Fig.3. The step of obtaining 102 the plurality of segments includes the sub-steps of recording 104 the signal with the accelerometer, and dividing 106 the recorded signal into the plurality of segments, for example by a technique as described in US 8235912 B2 and US 8469896 B2 relying on audible components of the accelerometer signal. The audible components are obtained by filtering the recorded signal.

In an alternative embodiment not covered by the claimed invention, an electrocardiography (ECG) signal is acquired simultaneously to the accelerometer signal, and the ECG signal is used for the segmentation of the latter. For example, a segmentation as described in Jensen et al. (Computing in Cardiology 2014; 41:29-32) can be used.

When obtaining 102 the plurality of segments, a method similar to the method described in Jensen et al. is employed to remove noisy segments. A high-pass filter with a lower cut-off of 65 Hz is applied to the segments and the onset of the first heart sound S1 is then determined by a known technique. Similarly, a high-pass filter with a lower cut-off of 50 Hz is applied to the segments and the onset of the second heart sound S2 is then determined by a known technique. The segments are then aligned according to the determined second heart sound S2. In alternative embodiment, the first heart sound is used instead.

The mean segment is determined 116 by summing the aligned segments to a single segment and dividing the resulting signal by the number of segments in the sum.

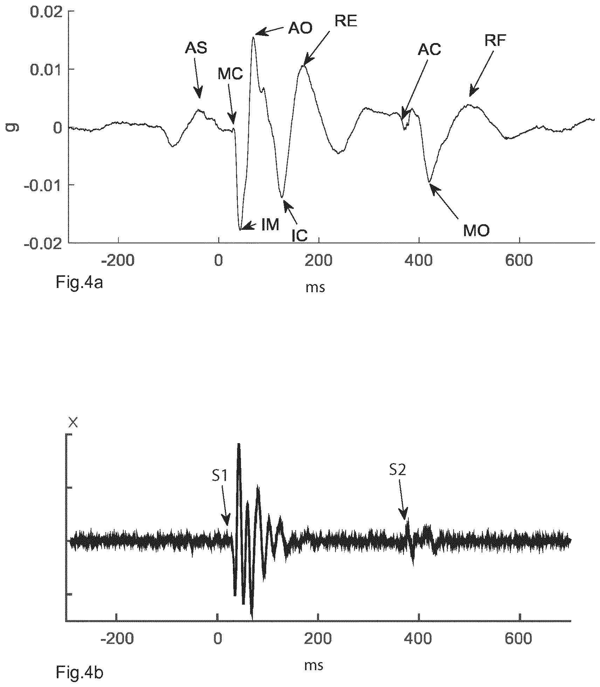

Fig.4a shows a segment that has been subjected to the above described band-pass filtering. The abscissa represents an acceleration in g (ms⁻²) and the ordinate the time in milliseconds (ms). Here, g is proportional to the voltage from the accelerometer 14. The zero point of the ordinate corresponds to the R peak in a simultaneously recorded segment of an ECG signal. A number of temporal features are indicated in Fig.4a, which are further described below.

Fig.4b shows a segment that has been subjected to a high-pass filter with a lower cut-off of 50 Hz, as described above. The abscissa represents the signal strength X (no unit) and the ordinate the time in milliseconds (ms). The latter has been aligned by the simultaneously recorded segment of an ECG signal in the same manner as described in relation to Fig.4a. The onset of the first heart sound (S1) and the second heart sound (S2) are indicated in Fig.4b.

In the steps of determining 118 the first temporal feature and determining 120 the second temporal feature 120 in the mean segment, the following temporal features are identified in the mean segment: the mitral valve closure (MC), isovolumic movement (MO), aortic valve opening (AO), the rapid ventricular ejection (RE), the aortic valve closure (AC), the mitral valve opening (MO), the rapid ventricular filling (RF), and the atrial systole (AS).

For example, with the first point in time determined to be the onset of the first heart sound (S1), see Fig.4b, the isovolumic movement (IM) is determined as the first local minima (IM) subsequent to the first point in time, the mitral valve closure (MC) is determined as the maximum negative deviation (MC) prior to the first local minima (IM) subsequent to the first point in time, the aortic valve opening (AO) is determined as the global maxima subsequent to the first point in time, and the rapid ventricular ejection (RE) is determined as the global maxima (RE) subsequent to the global minima (IC) subsequent to the global maxima (AO) subsequent to the first point in time. For example, the first point in time can be determined by a technology similar to the technology described in US 8235912 B2 and US 8469896 B2.

One measure that is determined is the amplitude or signal strength of the above mentioned temporal features. For example, the signal strengths of the following temporal features can be determined, the aortic valve opening (AO), the atrial systole (AS), the isometric contraction (IM), and the rapid ventricular ejection (RE) of a heart cycle.

Another measure that is determined is based on the locations in time, or positions on the abscissa of the Fig.4a, of a first temporal feature and a second temporal feature. The measure is then determined as the difference in time between a first temporal feature and a second temporal feature, wherein the measure is based on the determined difference. The following measures can be determined, the difference in time between the mitral valve closure (MC) and the rapid ventricular ejection (RE), the difference in time between the atrial systole (AS) and the mitral valve closure (MC), the difference in time between the aortic valve closing (AC) and the mitral valve opening (MO), the difference in time between the aortic valve opening (AO) and the aortic valve closing (AC), and the difference in time between the mitral valve closure (MC) and the aortic valve opening (AO).

Output is then provided 128 in the form of values that are displayed on an LCD display of the indicator 206, where the values represents the determined signal strengths and differences in time in the examples above.

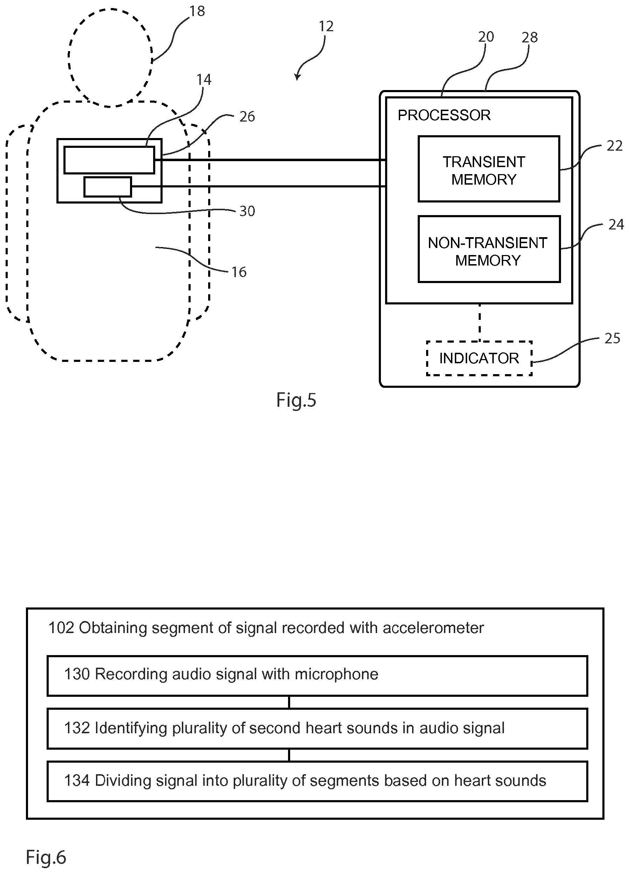

Fig.5 schematically illustrates an alternative embodiment of a system for quantifying heart failure. The system 12 is similar to the system described in relation to Fig.1 and features having the same or related functions have been given the same number indices. In addition, the system has a microphone 30 in the form of a transducer that can convert sound into an electrical signal. The microphone 30 is supported by the support 26.

The program code instructions in the non-transient memory 24 correspond to those described in relation to Figs. 2 and 3, but with different steps for obtaining 102 the plurality of segments, as illustrated in Fig.6. With the microphone placed on the chest of the person, the program code instructions additionally causes the processor 20 to operate the microphone 30 to record 130 an audio signal with the microphone 30 simultaneously to the signal being recorded 104 with the accelerometer 14. A plurality of second heart sounds (S2) are then identified 132 in the audio signal. The recorded signal is then divided 134 into the plurality of segments based on the time correlation between the signal and the audible signal, and the identified plurality of second heart sounds (S2) in the audible signal to obtain the plurality of segments. The identification of the second heart sounds (S2) and the segmentation is based on the technologies described in US 8235912 B2 and US 8469896 B2.

The subsequent alignment 108 is then also based on the plurality of second heart sounds (S2) determined from the audio signal.

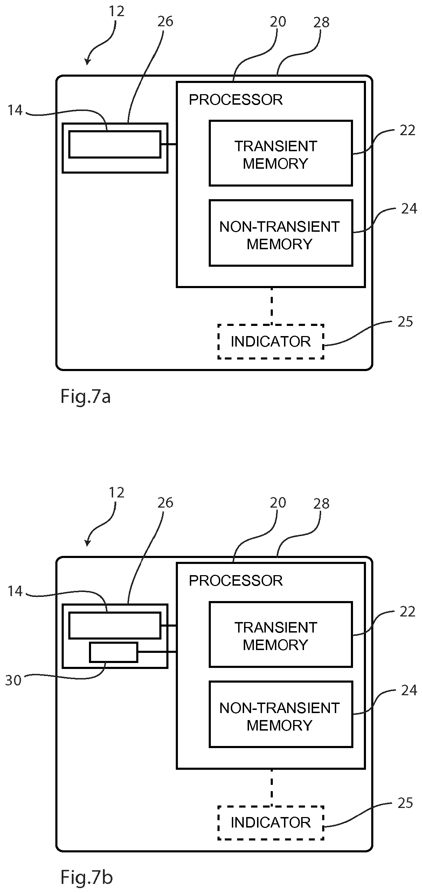

Fig.7a illustrates an alternative embodiment of the system 12 described in relation to Fig.1, with the only difference that the support 26 forms part of the housing 28 such that the housing 28 covers at least a portion of the accelerometer 14. In this embodiment, the housing 28 is placed on the chest of a person, which means that the accelerometer 14 is also placed on the chest of a person.

Similarly, Fig.7b illustrates an alternative embodiment of the system 12 described in relation to Fig. 5, with the only difference that the support 26 forms part of the housing 28 such that the housing 28 covers at least a portion of the accelerometer 14 and the microphone 30. In this embodiment, the housing 28 is placed on the chest of a person, which means that the accelerometer 14 and the microphone 30 are also placed on the chest of the person.

### Proof of concept

A custom lightweight 8 g piezoelectric accelerometer was developed for acquisition of the SCG signals. The low weight provides a better signal and the miniaturization allows for the accelerometer to be incorporated in another device. The accelerometer was used in a system as described above in relation to Figs.1-4.

It should be noted that the system identifies AO and MO and the resulting measures are not consistent. However, despite this disadvantage, AO and MO were used, since they have diagnostic importance.

The system was used on a first group of healthy subjects and on a second group of heart failure (HF) subjects undergoing pacemaker optimization. The majority of the heart failure subjects suffered from diastolic dysfunction. As is evident from Table 1 below, the amplitudes, or signal strength, and differences in time, or time intervals, of the temporal features provide a clear discrimination between heart failure patients and the normal subjects included.

**Table 1 shows average values and classification performance of the measures using the area under the receiving operating curve (AUC). The measures are sorted in decreasing classification performance.**

| Measure | Normal subjects Mean (STD) | HF subjects Mean (STD) | Classification AUC |
|---|---|---|---|
| Amplitudes | [mg] | [mg] | |
| AO | 21.9 (13.1) | 3.0 (2.2) | 98.0% |
| AS | 5.4 (2.5) | 1.5 (0.9) | 97.1% |
| IM | -23.7 (14.1) | -3.5 (3.5) | 95.0% |
| RE | 7.3 (4.1) | 2.6 (2.5) | 86.1% |

| Time intervals | [ms] | [ms] | |
|---|---|---|---|
| MC-RE | 140.6 (26.2) | 184.8 (27.8) | 89.5% |
| AS-MC | 65.6 (39.6) | 137.9 (71.1) | 83.4% |
| AC-MO | 43.1 (13.3) | 59.7 (24.8) | 74.6% |
| AO-AC | 296.6 (38.5) | 311.7 (57.2) | 63.2% |
| MC-AO | 41.7 (19.7) | 43.8 (31.8) | 42.2% |

Some of the measures of Table 1 were combined to improve the classification further. This exemplified in Tables 2-4

**Table 2 shows the combination of two measures, or three temporal features.**

| Amplitude | AUC |
|---|---|
| RE | 86.1% (73,4-98.8%) |

| Time interval | |
|---|---|
| MC-AC | 63.8% (47-80.6%) |

| Combined score | |
|---|---|
| RE + MC-AC | 90.7% (80-100%) |

**Table 3 shows the combination of three measures, or four temporal features.**

| Amplitude | AUC |
|---|---|
| RE | 86.1% (73.4-98.8%) |

| Time intervals | |
|---|---|
| AO-AC | 63.2% (46.3-80%) |
| MC-AC | 63.8% (47-80.6%) |

| Combined score | |
|---|---|
| RE + AO-AC + MC-AC | 92.8% (83.2-100%) |

**Table 4 shows the combination of four measures, or five temporal features.**

| Amplitude | AUC |
|---|---|
| RE | 86.1% (73.4-98.8%) |

| Time intervals | |
|---|---|
| AO-AC | 63.2% (46.3-80%) |
| MC-AC | 63.8% (47-80.6%) |
| AS-MC | 83.4% (69.8-97%) |

| Combined score | |
|---|---|
| RE + AO-AC + MC-AC + AS-MC | 95.1% (87.1-100%) |

### Feasible modifications of the Invention

The invention is not limited only to the embodiments described above in relation to the drawings, which primarily have an illustrative and exemplifying purpose. This patent application is intended to cover all adjustments and variants of the preferred embodiments described herein, thus the present invention is defined by the wording of the appended claims.

Thus, the equipment may be modified in all kinds of ways within the scope of the appended claims.

## Claims

1. A system (12) for quantifying heart failure, wherein the system (12) comprises:
(A) an accelerometer (14) configured to be placed on the chest of a person (18) for measuring accelerations and vibrations of the chest wall of the person (18) caused by myocardial movement,
(B) a processor (20) operatively connected to the accelerometer (14) and configured to:
- obtain (102) a plurality of segments of a signal recorded with the accelerometer (14),
- align (108) the plurality of segments, filter the plurality of segments with a band-pass filter having a lower cutoff frequency below 1 Hz and an upper cut-off frequency in the range 100-250 Hz, and determine a mean segment based on the plurality of segments,
- determine (118) a first temporal feature in the mean segment,
- determine (122) a measure based on at least one of the signal value, or amplitude, of the first temporal feature and the location in time of the first temporal feature, and
- provide (128) output information based on the determined measure,
**wherein** the processor (20) is further configured to:
- operate the accelerometer (14) to record the signal,
- filtering the signal to obtain an audio signal,
- identify a plurality of heart sounds in the audio signal, wherein each heart sound relates to a single cardiac cycle, and
- divide (106) the recorded signal into the plurality of segments based on the identified plurality of heart sounds to obtain the plurality of segments; or
**wherein** the system further comprises:
(C) a microphone (30) configured to be placed on the chest of the person (18) for measuring sounds generated by the beating heart, wherein the processor (20) is further operatively connected to the microphone (30) and configured to:
- operate the accelerometer (14) to record (104) the signal with the accelerometer (14),
- operate the microphone (30) to record an audio signal with the microphone simultaneously to the signal being recorded with the accelerometer (14),
- identify a plurality of heart sounds in the audio signal, wherein each heart sound relates to a single cardiac cycle, and
- divide (106) the recorded signal into the plurality of segments based on the identified plurality of heart sounds to obtain the plurality of segments.

2. The system according to claim 1, wherein aligning (108) the plurality of segments comprises: determining (110) the second heart sound (S2) in each of the plurality of segments, and aligning (112) the plurality of segments by the determined second heart sound (S2) of each segment.

3. The system according to claim 1 or 2, wherein the measure is based on the signal value, or amplitude, of the first temporal feature.

4. The system according to claim 3, wherein the first temporal feature corresponds to:
- the aortic valve opening (AO) of a heart cycle,
- the atrial systole (AS) of a heart cycle,
- the isometric contraction (IM) of a heart cycle, or
- the rapid ventricular ejection or rapid emptying event (RE) of a heart cycle.

5. The system according to any of the claims 1-4, wherein the processor (20) is further configured to:
- determining (120) a second temporal feature in the mean segment, and wherein
determining (122) a measure is further based on at least one of the signal value, or amplitude, of the second temporal feature and on the location in time of the second temporal feature.

6. The system according to claim 5, wherein the measure is based on the location in time of the first temporal feature and on the location in time of the second temporal feature, and wherein determining a measure (122) comprises: determining (126) the difference between the location in time of the first temporal feature and the location in time of the second temporal feature, and wherein the measure is based on the determined difference.

7. The system according to claim 5 or 6, wherein:
- the first temporal feature corresponds to the mitral valve closure (MC) and the second temporal feature corresponds to the rapid ventricular ejection or rapid emptying event (RE), or
- the first temporal feature corresponds to the atrial systole(AS) in the cardiac cycle and the second temporal feature corresponds to the mitral valve closure (MC) in the cardiac cycle, or
- the first temporal feature corresponds to the aortic valve closing (AC) and the second temporal feature corresponds to the mitral valve opening (MO), or
- the first temporal feature corresponds to the aortic valve opening (AO) and the second temporal feature corresponds to the aortic valve closing (AC), or
- the first temporal feature corresponds to the mitral valve closure (MC) and the second temporal feature corresponds to the aortic valve opening (AO).

8. The system according to any of the claims 1-7, wherein the processor (20) is further configured to: determining a first point in time in the mean segment corresponding to the onset of a heart sound (S1 or S2), and determining a first temporal feature and/or the second temporal feature further comprises: determining the first temporal feature and/or the second temporal feature relative to the first point in time.

9. The system according to any of the claims 1-8, wherein the lower cutoff frequency is below 0.5 Hz, 0.2 Hz, or approximately 0.1 Hz.

10. The system according to any of the claims 1-9, wherein the upper cutoff frequency is in the range 175-225 Hz, or approximately 200 Hz, or in one of the ranges 100-150 Hz, 150-200 Hz, and 200-250 Hz.

11. A computer program product for being used in a system (12) for quantifying heart failure comprising: (A) an accelerometer (14) for being placed on the chest of a person (18) for measuring accelerations and vibrations of the chest wall of the person (18) caused by myocardial movement, optionally a microphone (30) configured to be placed on the chest of the person (18) for measuring sounds generated by the beating heart, and
(B) a processor (20) operatively connected with the accelerometer (14) and optionally the microphone (30), the computer program product comprising
program code instructions configured to, when executed by the processor (20) of the system (12), cause the processor (20) to:
- obtain a signal with the accelerometer (14) and form a plurality of segments from the signal, wherein each segment covers, or corresponds to, a cardiac cycle.
- record a plurality of segments of a signal with the accelerometer (14),
- align the plurality of segments, filter the plurality of segments with a band-pass filter having a lower cutoff frequency below 1 Hz and an upper cut-off frequency in the range 100-250 Hz, and determine a mean segment based on the plurality of segments,
- determine a first temporal feature in the mean segment,
- determine a measure based on at least one of the signal value, or amplitude, of the first temporal feature and the location in time of the first temporal feature, and
- provide output information based on the determined measure,
**wherein** the computer program product further comprises program code instructions configured to, when executed by the processor (20) of the system (12), cause the processor (20) to:
- operate the accelerometer (14) to record the signal,
- filtering the signal to obtain an audio signal,
- identify a plurality of heart sounds in the audio signal, wherein each heart sound relates to a single cardiac cycle, and
- divide (106) the recorded signal into the plurality of segments based on the identified plurality of heart sounds to obtain the plurality of segments; or **wherein** the computer program product further comprises program code instructions configured to, when executed by the processor (20) of the system (12), cause the processor (20) to:
- operate the accelerometer (14) to record (104) the signal with the accelerometer (14),
- operate the microphone (30) to record an audio signal with the microphone simultaneously to the signal being recorded with the accelerometer (14),
- identify a plurality of heart sounds in the audio signal, wherein each heart sound relates to a single cardiac cycle, and
- divide (106) the recorded signal into the plurality of segments based on the identified plurality of heart sounds to obtain the plurality of segments.

12. A non-transient memory (24) on which a computer program product according to claim 11 is stored.

## Patentansprüche

1. System (12) zum Quantifizieren von Herzinsuffizienz, wobei das System (12) Folgendes umfasst:
(A) einen Beschleunigungsmesser (14), der dazu konfiguriert ist, auf der Brust einer Person (18) platziert zu werden, um Beschleunigungen und Vibrationen der Brustwand der Person (18) zu messen, die durch Myokardbewegungen hervorgerufen werden,
(B) einen Prozessor (20), der betriebsfähig mit dem Beschleunigungsmesser (14) verbunden und zu Folgendem konfiguriert ist:
Erlangen (102) einer Vielzahl von Segmenten eines mit dem Beschleunigungsmesser (14) aufgezeichneten Signals,
Ausrichten (108) der Vielzahl von Segmenten, Filtern der Vielzahl von Segmenten mit einem Bandpassfilter mit einer unteren Grenzfrequenz unter 1 Hz und einer oberen Grenzfrequenz im Bereich von 100-250 Hz, und Ermitteln eines mittleren Segments basierend auf der Vielzahl von Segmenten,
Ermitteln (118) eines ersten zeitlichen Merkmals in dem mittleren Segment,
Ermitteln (122) eines Maßes basierend auf mindestens einem von dem Signalwert oder der Amplitude des ersten zeitlichen Merkmals und der zeitlichen Position des ersten zeitlichen Merkmals, und Bereitstellen (128) von Ausgabeinformationen basierend auf dem ermittelten Maß,
wobei der Prozessor (20) ferner zu Folgendem konfiguriert ist:
Betätigen des Beschleunigungsmessers (14), um das Signal aufzuzeichnen,
Filtern des Signals, um ein Audiosignal zu erlangen,
Identifizieren einer Vielzahl von Herztönen in dem Audiosignal,
wobei sich jeder Herzton auf einen einzelnen Herzzyklus bezieht, und
Teilen (106) des aufgezeichneten Signals in die Vielzahl von Segmenten basierend auf der identifizierten Vielzahl von Herztönen, um die Vielzahl von Segmenten zu erlangen; oder
wobei das System ferner Folgendes umfasst:
(C) ein Mikrofon (30), das dazu konfiguriert ist, auf der Brust der Person (18) platziert zu werden, um die von dem schlagenden Herzen erzeugten Töne zu messen, wobei der Prozessor (20) ferner betriebsfähig mit dem Mikrofon (30) verbunden und zu Folgendem konfiguriert ist:
Betätigen des Beschleunigungsmessers (14), um das Signal mit dem Beschleunigungsmesser (14) aufzuzeichnen (104),
Betätigen des Mikrofons (30), um gleichzeitig mit dem Signal, das mit dem Beschleunigungsmesser (14) aufgezeichnet wird, ein Audiosignal mit dem Mikrofon aufzuzeichnen,
Identifizieren einer Vielzahl von Herztönen in dem Audiosignal,
wobei sich jeder Herzton auf einen einzelnen Herzzyklus bezieht, und
Teilen (106) des aufgezeichneten Signals in die Vielzahl von Segmenten basierend auf der identifizierten Vielzahl von Herztönen, um die Vielzahl von Segmenten zu erlangen.

2. System nach Anspruch 1, wobei Ausrichten (108) der Vielzahl von Segmenten Folgendes umfasst: Ermitteln (110) des zweiten Herztons (S2) in jedem der Vielzahl von Segmenten und Ausrichten (112) der Vielzahl von Segmenten nach dem ermittelten zweiten Herzton (S2) jedes Segments.

3. System nach Anspruch 1 oder 2, wobei die Messung auf dem Signalwert oder der Amplitude des ersten zeitlichen Merkmals basiert.

4. System nach Anspruch 3, wobei das erste zeitliche Merkmal Folgendem entspricht:
der Aortenklappenöffnung (AO) eines Herzzyklus,
der Vorhofsystole (AS) eines Herzzyklus,
der isometrischen Kontraktion (IM) eines Herzzyklus oder
der schnellen ventrikulären Ejektion oder dem schnellen Entleerungsereignis (RE) eines Herzzyklus.

5. System nach einem der Ansprüche 1-4, wobei der Prozessor (20) ferner zu Folgendem konfiguriert ist:
Ermitteln (120) eines zweiten zeitlichen Merkmals in dem mittleren Segment, und wobei
Ermitteln (122) eines Maßes ferner auf mindestens einem von dem Signalwert oder der Amplitude des zweiten zeitlichen Merkmals und der zeitlichen Position des zweiten zeitlichen Merkmals basiert.

6. System nach Anspruch 5, wobei das Maß auf der zeitlichen Position des ersten zeitlichen Merkmals und auf der zeitlichen Position des zweiten zeitlichen Merkmals basiert und wobei Ermitteln eines Maßes (122) Folgendes umfasst: Ermitteln (126) der Differenz zwischen der zeitlichen Position des ersten zeitlichen Merkmals und der zeitlichen Position des zweiten zeitlichen Merkmals, und wobei das Maß auf der ermittelten Differenz basiert.

7. System nach Anspruch 5 oder 6, wobei:
das erste zeitliche Merkmal dem Mitralklappenverschluss (MC) entspricht und das zweite zeitliche Merkmal der schnellen ventrikulären Ejektion oder dem schnellen Entleerungsereignis (RE) entspricht, oder
das erste zeitliche Merkmal der Vorhofsystole (AS) in dem Herzzyklus entspricht und das zweite zeitliche Merkmal dem Mitralklappenverschluss (MC) in dem Herzzyklus entspricht oder das erste zeitliche Merkmal dem Schließen der Aortenklappe (AC) entspricht und das zweite zeitliche Merkmal dem Öffnen der Mitralklappe (MO) entspricht, oder
das erste zeitliche Merkmal dem Öffnen der Aortenklappe (AO) entspricht und das zweite zeitliche Merkmal dem Schließen der Aortenklappe (AC) entspricht, oder
das erste zeitliche Merkmal dem Mitralklappenverschluss (MC) entspricht und das zweite zeitliche Merkmal dem Öffnen der Aortenklappe (AO) entspricht.

8. System nach einem der Ansprüche 1-7, wobei der Prozessor (20) ferner zu Folgendem konfiguriert ist: Ermitteln eines ersten Zeitpunkts in dem mittleren Segment, der dem Einsetzen eines Herztons (S1 oder S2) entspricht, und Ermitteln eines ersten zeitlichen Merkmals und/oder das zweite zeitliche Merkmal ferner umfasst: Ermitteln des ersten zeitlichen Merkmals und/oder des zweiten zeitlichen Merkmals bezogen auf den ersten Zeitpunkt.

9. System nach einem der Ansprüche 1-8, wobei die untere Grenzfrequenz unter 0,5 Hz, 0,2 Hz oder ungefähr 0,1 Hz liegt.

10. System nach einem der Ansprüche 1-9, wobei die obere Grenzfrequenz in dem Bereich von 175-225 Hz oder ungefähr 200 Hz oder in einem der Bereiche 100-150 Hz, 150-200 Hz und 200-250 Hz liegt.

11. Computerprogrammprodukt zur Verwendung in einem System (12) zum Quantifizieren von Herzinsuffizienz, umfassend: (A) einen Beschleunigungsmesser (14) zum Platzieren auf der Brust einer Person (18), um Beschleunigungen und Vibrationen der Brustwand der Person (18) zu messen, die durch eine Myokardbewegung hervorgerufen werden, optional ein Mikrofon (30), das dazu konfiguriert ist, auf der Brust der Person (18) platziert zu werden, um Töne zu messen, die durch das schlagende Herz erzeugt werden, und (B) einen Prozessor (20), der betriebsfähig mit dem Beschleunigungsmesser (14) und optional dem Mikrofon (30) verbunden ist, wobei das Computerprogrammprodukt Programmcodeanweisungen umfasst, die dazu konfiguriert sind, bei Ausführung durch den Prozessor (20) des Systems (12) den Prozessor (20) zu Folgendem zu veranlassen:
Erlangen eines Signals mit dem Beschleunigungsmesser (14) und Bilden einer Vielzahl von Segmenten aus dem Signal, wobei jedes Segment einen Herzzyklus abdeckt oder einem Herzzyklus entspricht.
Aufzeichnen einer Vielzahl von Segmenten eines Signals mit dem Beschleunigungsmesser (14),
Ausrichten der Vielzahl von Segmenten, Filtern der Vielzahl von Segmenten mit einem Bandpassfilter mit einer unteren Grenzfrequenz unter 1 Hz und einer oberen Grenzfrequenz im Bereich von 100-250 Hz, und Ermitteln eines mittleren Segments basierend auf der Vielzahl von Segmenten,
Ermitteln eines ersten zeitlichen Merkmals in dem mittleren Segment,
Ermitteln eines Maßes basierend auf mindestens einem von dem Signalwert oder der Amplitude des ersten zeitlichen Merkmals und der zeitlichen Position des ersten zeitlichen Merkmals, und
Bereitstellen von Ausgabeinformationen basierend auf dem ermittelten Maß, wobei das Computerprogrammprodukt ferner Programmcodeanweisungen umfasst, die dazu konfiguriert sind, bei Ausführung durch den Prozessor (20) des Systems (12) den Prozessor (20) zu Folgendem zu veranlassen:
Betätigen des Beschleunigungsmessers (14), um das Signal aufzuzeichnen,
Filtern des Signals, um ein Audiosignal zu erlangen,
Identifizieren einer Vielzahl von Herztönen in dem Audiosignal,
wobei sich jeder Herzton auf einen einzelnen Herzzyklus bezieht, und
Teilen (106) des aufgezeichneten Signals in die Vielzahl von Segmenten basierend auf der identifizierten Vielzahl von Herztönen, um die Vielzahl von Segmenten zu erlangen; oder wobei das Computerprogrammprodukt ferner Programmcodeanweisungen umfasst, die dazu konfiguriert sind, bei Ausführung durch den Prozessor (20) des Systems (12) den Prozessor (20) zu Folgendem zu veranlassen:
Betätigen des Beschleunigungsmessers (14), um das Signal mit dem Beschleunigungsmesser (14) aufzuzeichnen (104),
Betätigen des Mikrofons (30), um gleichzeitig mit dem Signal, das mit dem Beschleunigungsmesser (14) aufgezeichnet wird, ein Audiosignal mit dem Mikrofon aufzuzeichnen,
Identifizieren einer Vielzahl von Herztönen in dem Audiosignal,
wobei sich jeder Herzton auf einen einzelnen Herzzyklus bezieht, und
Teilen (106) des aufgezeichneten Signals in die Vielzahl von Segmenten basierend auf der identifizierten Vielzahl von Herztönen, um die Vielzahl von Segmenten zu erlangen.

12. Nichtflüchtiger Speicher (24), auf dem ein Computerprogrammprodukt nach Anspruch 11 gespeichert ist.

## Revendications

1. Système (12) pour quantifier l'insuffisance cardiaque, le système (12) comprenant :
(A) un accéléromètre (14) configuré pour être placé sur la poitrine d'une personne (18) pour mesurer les accélérations et les vibrations de la paroi thoracique de la personne (18) provoquées par le mouvement du myocarde,
(B) un processeur (20) relié fonctionnellement à l'accéléromètre (14) et configuré pour :
- obtenir (102) une pluralité de segments d'un signal enregistré avec l'accéléromètre (14),
- aligner (108) la pluralité de segments, filtrer la pluralité de segments avec un filtre passe-bande ayant une fréquence de coupure inférieure inférieure à 1 Hz et une fréquence de coupure supérieure dans la plage de 100 à 250 Hz, et déterminer un segment moyen sur la base de la pluralité de segments,
- déterminer (118) une première caractéristique temporelle dans le segment moyen,
- déterminer (122) une mesure basée sur au moins l'une de la valeur du signal, ou de l'amplitude, de la première caractéristique temporelle et la localisation dans le temps de la première caractéristique temporelle, et
- fournir (128) des informations de sortie basées sur la mesure déterminée,
dans lequel le processeur (20) est également configuré pour :
- faire fonctionner l'accéléromètre (14) pour enregistrer le signal,
- filtrer le signal pour obtenir un signal audio,
- identifier une pluralité de bruits cardiaques dans le signal audio, où chaque bruit cardiaque se rapporte à un cycle cardiaque unique, et
- diviser (106) le signal enregistré en la pluralité de segments sur la base de la pluralité identifiée de bruits cardiaques pour obtenir la pluralité de segments ; ou
le système comprenant également :
(C) un microphone (30) configuré pour être placé sur la poitrine de la personne (18) pour mesurer les bruits générés par les battements du coeur, où le processeur (20) est également relié de manière fonctionnelle au microphone (30) et configuré pour :
- faire fonctionner l'accéléromètre (14) pour enregistrer (104) le signal avec l'accéléromètre (14),
- faire fonctionner le microphone (30) pour enregistrer un signal audio avec le microphone simultanément au signal en cours d'enregistrement avec l'accéléromètre (14),
- identifier une pluralité de bruits cardiaques dans le signal audio, où chaque bruit cardiaque se rapporte à un cycle cardiaque unique, et
- diviser (106) le signal enregistré en la pluralité de segments sur la base de la pluralité identifiée de bruits cardiaques pour obtenir la pluralité de segments.

2. Système selon la revendication 1, dans lequel l'alignement (108) de la pluralité de segments comprend : la détermination (110) du second bruit cardiaque (S2) dans chacun de la pluralité de segments, et l'alignement (112) de la pluralité de segments par le second bruit cardiaque (S2) déterminé de chaque segment.

3. Système selon la revendication 1 ou 2, dans lequel la mesure est basée sur la valeur du signal, ou l'amplitude, de la première caractéristique temporelle.

4. Système selon la revendication 3, dans lequel la première caractéristique temporelle correspond à :
- l'ouverture de la valve aortique (AO) d'un cycle cardiaque,
- la systole atriale (SA) d'un cycle cardiaque,
- la contraction isométrique (IM) d'un cycle cardiaque, ou
- l'éjection ventriculaire rapide ou l'événement de vidange rapide (RE) d'un cycle cardiaque.

5. Système selon l'une quelconque des revendications 1 à 4, dans lequel le processeur (20) est également configuré pour :
- déterminer (120) une seconde caractéristique temporelle dans le segment moyen, et dans lequel
la détermination (122) d'une mesure est également basée sur au moins l'une de la valeur du signal, ou de l'amplitude, de la seconde caractéristique temporelle et sur la localisation dans le temps de la seconde caractéristique temporelle.

6. Système selon la revendication 5, dans lequel la mesure est basée sur la localisation dans le temps de la première caractéristique temporelle et sur la localisation dans le temps de la seconde caractéristique temporelle, et dans lequel la détermination d'une mesure (122) comprend : la détermination (126) de la différence entre la localisation dans le temps de la première caractéristique temporelle et la localisation dans le temps de la seconde caractéristique temporelle, et dans lequel la mesure est basée sur la différence déterminée.

7. Système selon la revendication 5 ou 6, dans lequel :
- la première caractéristique temporelle correspond à la fermeture de la valve mitrale (MC) et la seconde caractéristique temporelle correspond à l'éjection ventriculaire rapide ou à l'événement de vidange rapide (RE), ou
- la première caractéristique temporelle correspond à la systole atriale (AS) dans le cycle cardiaque et la seconde caractéristique temporelle correspond à la fermeture de la valve mitrale (MC) dans le cycle cardiaque, ou
- la première caractéristique temporelle correspond à la fermeture de la valve aortique (AC) et la seconde caractéristique temporelle correspond à l'ouverture de la valve mitrale (MO), ou
- la première caractéristique temporelle correspond à l'ouverture de la valve aortique (AO) et la seconde caractéristique temporelle correspond à la fermeture de la valve aortique (AC), ou
- la première caractéristique temporelle correspond à la fermeture de la valve mitrale (MO) et la seconde caractéristique temporelle correspond à l'ouverture de la valve aortique (AO).

8. Système selon l'une quelconque des revendications 1 à 7, dans lequel le processeur (20) est également configuré pour : déterminer un premier instant dans le segment moyen correspondant à l'apparition d'un bruit cardiaque (S1 ou S2), et la détermination d'une première caractéristique temporelle et/ou de la seconde caractéristique temporelle comprend également : la détermination de la première caractéristique temporelle et/ou de la seconde caractéristique temporelle par rapport au premier instant.

9. Système selon l'une quelconque des revendications 1 à 8, dans lequel la fréquence de coupure inférieure est inférieure à 0,5 Hz, à 0,2 Hz ou à environ 0,1 Hz.

10. Système selon l'une quelconque des revendications 1 à 9, dans lequel la fréquence de coupure supérieure est dans la plage de 175 à 225 Hz, ou approximativement 200 Hz, ou dans l'une des plages de 100 à 150 Hz, 150 à 200 Hz et 200 Hz à 250 Hz.

11. Produit-programme informatique destiné à être utilisé dans un système (12) pour quantifier l'insuffisance cardiaque comprenant : (A) un accéléromètre (14) destiné à être placé sur la poitrine d'une personne (18) pour mesurer les accélérations et les vibrations de la paroi thoracique de la personne (18) provoquées par le mouvement du myocarde, éventuellement un microphone (30) configuré pour être placé sur la poitrine de la personne (18) pour mesurer les bruits générés par les battements du coeur, et (B) un processeur (20) relié de manière fonctionnelle à l'accéléromètre (14) et éventuellement au microphone (30), le produit-programme informatique comprenant des instructions de code de programme configurées pour, lorsqu'elles sont exécutées par le processeur (20) du système (12), amener le processeur (20) à :
- obtenir un signal avec l'accéléromètre (14) et former une pluralité de segments à partir du signal, chaque segment couvrant, ou correspondant à, un cycle cardiaque.
- enregistrer une pluralité de segments d'un signal avec l'accéléromètre (14),
- aligner la pluralité de segments, filtrer la pluralité de segments avec un filtre passe-bande ayant une fréquence de coupure inférieure inférieure à 1 Hz et une fréquence de coupure supérieure dans la plage de 100 à 250 Hz, et déterminer un segment moyen sur la base de la pluralité de segments,
- déterminer une première caractéristique temporelle dans le segment moyen,
- déterminer une mesure basée sur au moins l'une de la valeur du signal, ou de l'amplitude, de la première caractéristique temporelle et la localisation dans le temps de la première caractéristique temporelle, et
- fournir des informations de sortie sur la base de la mesure déterminée, le produit-programme informatique comprenant également des instructions de code de programme configurées pour, lorsqu'elles sont exécutées par le processeur (20) du système (12), amener le processeur (20) à :
- faire fonctionner l'accéléromètre (14) pour enregistrer le signal,
- filtrer le signal pour obtenir un signal audio,
- identifier une pluralité de bruits cardiaques dans le signal audio, où chaque bruit cardiaque se rapporte à un cycle cardiaque unique, et
- diviser (106) le signal enregistré en la pluralité de segments sur la base de la pluralité identifiée de bruits cardiaques pour obtenir la pluralité de segments ; ou dans lequel le produit-programme informatique comprenant également des instructions de code de programme configurées pour, lorsqu'elles sont exécutées par le processeur (20) du système (12), amener le processeur (20) à :
- faire fonctionner l'accéléromètre (14) pour enregistrer (104) le signal avec l'accéléromètre (14),
- faire fonctionner le microphone (30) pour enregistrer un signal audio avec le microphone simultanément au signal en cours d'enregistrement avec l'accéléromètre (14),
- identifier une pluralité de bruits cardiaques dans le signal audio, où chaque bruit cardiaque se rapporte à un cycle cardiaque unique, et
- diviser (106) le signal enregistré en la pluralité de segments sur la base de la pluralité identifiée de bruits cardiaques pour obtenir la pluralité de segments.

12. Mémoire non transitoire (24) sur laquelle un produit-programme informatique selon la revendication 11 est stocké.
